# EUROPEAN PATENT SPECIFICATION

(11) **EP 2 398 885 B1**
(45) Date of publication and mention of the grant of the patent: **28.12.2016**
(21) Application number: 10744129.7
(22) Date of filing: 04.02.2010
(51) Int. Cl.: A61Q 5/02, A61Q 17/04, A61Q 19/10, A61K 8/14, A61K 8/35, A61K 8/39, A61K 8/40, A61K 8/42, A61K 8/44, A61K 8/46, A61K 8/86

(54) **PERSONAL CLEANSING COMPOSITION INCLUDING A STRUCTURED SURFACTANT SYSTEM AND A SUN PROTECTION FACTOR COMPOSITION**
KÖRPERREINIGUNGSZUSAMMENSETZUNG MIT EINEM STRUKTURIERTEN TENSIDSYSTEM UND SONNENSCHUTZFAKTORZUSAMMENSETZUNG
COMPOSITION D'HYGIÈNE PERSONNELLE COMPRENANT UN SYSTÈME DE TENSIO-ACTIF STRUCTURÉ ET UNE COMPOSITION DE FACTEUR DE PROTECTION SOLAIRE

(30) Priority: 19.02.2009 US 389044
(43) Date of publication of application: 28.12.2011
(73) Proprietor: The Dial Corporation, Scottsdale, AZ 85255 (US)
(72) Inventor: GE, Haiyan, Scottsdale Arizona 85254 (US); DOERING, Thomas, Scottsdale Arizona 85255 (US); SEITZ, JR., Earl P., Scottsdale Arizona 85260 (US)
(74) Representative: Henkel IP Department
(86) International application number: PCT/US2010/023136
(87) International publication number: WO 2010/096275

(56) References cited:
- US-A- 5 674 511
- US-A- 5 904 917
- US-A1- 2004 002 438
- US-A1- 2005 233 935
- US-A1- 2005 265 936
- US-A1- 2006 128 579
- US-A1- 2007 207 936
- US-B1- 6 224 852
- US-B1- 6 576 228
- US-B1- 6 998 113

## Description

### TECHNICAL FIELD

The present invention generally relates to personal cleansing compositions, and more particularly relates to such compositions that include sunscreen products.

### BACKGROUND

Exposure to ultraviolet light, primarily through exposure to solar radiation, produces a number of harmful effects including premature skin aging, loss of elasticity, wrinkling, drying, and an increased risk of developing skin cancer. Numerous sunscreen products arc currently manufactured and marketed to protect against such harmful effects. Sunscreen products contain agents that are known to filter out some of the sun's harmful radiation, and are incorporated into various base creams, ointments, lotions, solutions, or suspensions. The sunscreen formulations are typically applied just prior to anticipated exposure to solar radiation to provide short term protection, and are thereafter removed by bathing, washing or normal desquamation of skin.

Soap has long been used to remove oil from the skin exterior surface. More particularly, soaps in various formulations including bars and body washes include surfactants that may have associated charges, which in many soap formulations are both positive and negative. Charged and uncharged surfactants are used in combination to attract and remove from the skin the various oils accumulated thereon. Attempts have been made to combine sunscreen compositions with soaps, but to date none has effectively provided sufficient deposition of sunscreen compounds to provide a highly beneficial and sustained sun protection factor (SPF) to the skin. Furthermore, in many cases phase separation is experienced over time in cleansing formulations that contain sunscreen compositions.

Accordingly, it is desirable to provide a stable personal cleansing composition that includes sunscreen compositions. In addition, it is desirable to include additional components in the personal cleansing composition that will enhance the sustained effect of the sunscreen compositions on the skin. Furthermore, other desirable features and characteristics of the present invention will become apparent from the subsequent detailed description and the appended claims, taken in conjunction with the accompanying drawings and the foregoing technical field and background.

### BRIEF SUMMARY

A personal cleansing composition is provided, which includes a structured surfactant system including a blend of surfactants that form spherulite structures in an aqueous medium. The composition also includes water, a solubilizer and at least one sunscreen
compound. The personal cleansing composition deposits the at least one sunscreen compound onto skin in a manner whereby the sunscreen compound imparts to the skin a sun protection factor of at least 4 after rinsing the personal cleansing composition.

A composition for use in a method of cleansing and providing sun protection factor to skin is also provided. The homogenous and shelf stable personal cleansing composition described above is applied to the skin, and is then rinsed from the skin in a manner whereby a sufficient amount of the at least one sunscreen compound remains deposited onto skin to impart to the skin a sun protection factor of at least 4 after rinsing the personal cleansing composition.

### DETAILED DESCRIPTION

The following detailed description is merely exemplary in nature and is not intended to limit the invention or the application and uses of the invention. Furthermore, there is no intention to be bound by any expressed or implied theory presented in the preceding technical field, background, brief summary or the following detailed description.

According to an exemplary embodiment of the invention, a personal cleansing composition includes a spherulite structured surfactant system in order to stably incorporate an effective combination ofUVA and UVB sunscreens. The composition is able to perform the traditional functions of soap and/or surfactant formulations. Such functions include the removal of contaminants and oil from the skin exterior surface, the plentiful generation of lather, and the provision of a good skin feel after rinsing the composition from the skin surface. Additionally, the personal care composition combines UVA and UVB sunscreen compounds with soaps and/or surfactant blends in a manner whereby the sunscreen compounds are sufficiently deposited on the skin surface to provide a highly beneficial and sustained SPF of 4 to 8 to the skin after rinsing the personal cleansing composition. Furthermore, the personal care composition is stable over extended time and changes in temperature. Consequently, phase separation due to coalescence of sunscreen compounds is not experienced.

As used herein, a spherulite structured surfactant system is a composition that includes a plurality of spherical bilayers of surfactant arranged in parallel and separated by liquid medium. More particularly, spherulites are vesicles formed from a number of concentric spheroidal shells, each of which is a bilayer of surfactant. The spherulites are typically between 0.1 and 50 microns in diameter, more commonly between 1 and 10 microns in diameter, and so differ fundamentally from micelles. Unlike micellar solutions, spherulitic compositions are typically anisotropic and non-Newtonian. When closely packed, spherulites have good solid suspending properties and are capable of suspending discontinuous water insoluble or partially water soluble solids, liquids and/or gases.

At least one suitable anionic surfactant is necessary to form the spherulite structured surfactant composition. Any anionic surfactant that is acceptable for use in the intended end use application may be used as the anionic surfactant component, including, for example, alkyl sulfonates, alkyl alkoxy carboxylates, alkyl alkoxylated sulfates, monoalkyl phosphates, dialkyl phosphates, linear alkylbenzene sulfonates, alpha olefin sulfonates, paraffin sulfonates, alkyl ester sulfonates, alkyl sulfates, alkyl alkoxy sulfates, sarcosinates, sulfosuccinates, isethionates, and taurates, as well as mixtures thereof. Commonly used anionic surfactants that are suitable as the anionic surfactant component of the composition of the present invention include, for example, ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium-monoalkyl phosphates, sodium dialkyl phosphates, sodium lauroyl sarcosinate, lauroyl sarcosine, cocoyl sarcosine, ammonium cocyl sulfate, ammonium lauryl sulfate, sodium cocyl sulfate, sodium trideceth sulfate, sodium tridecyl sulfate, ammonium trideceth sulfate, ammonium tridecyl sulfate, sodium cocoyl isethionate, disodium laureth sulfosuccinate, sodium methyl oleoyl taurate, sodium laureth carboxylate, sodium trideceth carboxylate, sodium lauryl sulfate, potassium cocyl sulfate, potassium lauryl sulfate, monoethanolamine cocyl sulfate, sodium tridecyl benzene sulfonate, and sodium dodecyl benzene sulfonate. Branched anionic surfactants are particularly preferred, such as sodium trideceth sulfate, sodium tridecyl sulfate, ammonium trideceth sulfate, ammonium tridecyl sulfate, and sodium trideceth carboxylate.

The cation of any anionic surfactant is typically sodium but may alternatively be potassium, lithium, calcium, magnesium, ammonium, or an alkyl ammonium having up to 6 aliphatic carbon atoms including isopropylammonium, monoethanolammonium, diethanolammonium, and triethanolammonium. Ammonium and ethanolammonium salts are generally more soluble that the sodium salts. Mixtures of the above cations may be used.

An exemplary composition includes the anionic surfactant at a concentration of from 3 to 40 wt.%, more typically from 5 to 30 wt.%, and still more typically from 8 to 20 wt.%.

The structured surfactant composition of the present invention may optionally further comprise, in addition to the anionic surfactant, one or more cationic surfactants, one or more additional non-ionic surfactants, one or more zwitterionic surfactants, and/or one or more amphoteric surfactants. Any of such optional components may be present at an effective concentration to function as a structurant for the anionic surfactant. An exemplary composition includes one or more amphoteric surfactant, along with one or more cationic surfactants and/or non-ionic surfactants, such as fatty alcohols, ethoxylated alcohols, and fatty acids, at an effective concentration to act as structurants for anionic surfactants. According to one exemplary embodiment, the spherulite structured system includes any surfactant blend that is manufactured by Rhodia and sold under the trademark Miracare^{®} SLB. A particular system includes the surfactant blend sold under the trademark Miracare^{®} SLB-365™, which includes a mixture of anionic surfactant sodium tridcceth sulfate, amphoteric surfactant sodium lauroamphoacetate, and nonionic surfactant cocomonoethanolamide.

Typically, the greater the amount of anionic surfactant present in relation to its solubility, the lesser the amount of structurant required in order to form a structure capable of supporting the combined UVA and UVB sunscreen compounds materials. The structurant is incorporated in an amount sufficient to promote the structured surfactant composition and may be added separately or may be included in one of the other raw materials added to the composition. An exemplary composition includes the structured surfactant composition at up to about 40 wt.%, more preferably from 10 to 40 wt.%, and most preferably from 30 to 40 wt.%.

Examples of quaternary ammonium compounds include those of the monoalkyl amine derivative type, dialkyl amine derivative type, and imidazoline derivative type. Examples of the monoalkyl amine derivative type include cetyl trimethyl ammonium bromide (also known as CETAB or cetrimonium bromide), cetyl trimethyl ammonium chloride (also known as cetrimonium chloride), myristyl trimethyl ammonium bromide (also known as myrtrimonium bromide or Quaternium-13), stearyl dimethyl benzyl ammonium chloride (also known as stearalkonium chloride), oleyl dimethyl benzyl ammonium chloride, (also known as olealkonium chloride), lauryl/myristryl trimethyl ammonium methosulfate (also known as cocotrimonium methosulfate), cetyl-dimethyl-(2)hydroxyethyl ammonium dihydrogen phosphate (also known as hydroxyethyl cetyldimonium phosphate), bassuamidopropylkonium chloride, cocotrimonium chloride, distearyldimonium chloride, wheat germ-amidopropalkonium chloride, stearyl octyldimonium methosulfate, isostearaminopropal-konium chloride, dihydroxypropyl PEG-5 linoleaminium chloride, PEG-2 stearmonium chloride, Quaternium 18, Quaternium 80, Quaternium 82, Quaternium 84, behentrimonium chloride, dicetyl dimonium chloride, behentrimonium methosulfate, tallow trimonium chloride and behenamidopropyl ethyl dimonium ethosulfate. Examples of the dialkyl amine derivative type include distearyldimonium chloride, dicetyl dimonium chloride, stearyl octyldimonium methosulfate, dihydrogenated palmoylethyl hydroxyethylmonium methosulfate, dipalmitoylethyl hydroxyethylmonium methosulfate, dioleoylethyl hydroxyethylmonium methosulfate, hydroxypropyl bisstearyldimonium chloride, and mixtures thereof. Examples of the imidazoline derivative type include isostearyl benzylimidonium chloride, cocoyl benzyl hydroxyethyl imidazolinium chloride, cocoyl hydroxyethylimidazolinium PG-chloride phosphate, Quaternium 32. and stearyl hydroxyethylimidonium chloride, and mixtures thereof.

The nonionic surfactants may be compounds produced by the condensation of alkylene oxide groups with an organic hydrophobic compound, which may be aliphatic or alkyl aromatic in nature. Examples of useful nonionic surfactants include the polyethylene, polypropylene, and polybutylene oxide condensates of alkyl phenols, fatty acid amide surfactants, polyhydroxy fatty acid amide surfactants, alkyl ethoxylate surfactants, alkanoyl glucose amide surfactants, and alkylpolyglycosides. Specific examples of suitable nonionic surfactants include alkanolamides such as cocamide DEA, cocamide MEA, cocamide MIPA, lauramide DEA, and lauramide MEA, sorbitan laurate, sorbitan distearate, fatty acids or fatty acid esters such as lauric acid, and isostearic acid, fatty alcohols or ethoxylated fatty alcohols such as lauryl alcohol, laureth-4, laureth-7, laureth-9, laureth-40, trideceth alcohol, C11-15 pareth-9, C12-13 Pareth-3, and C14-15 Pareth-11, alkylpolyglucosides such as decyl glucoside, lauryl glucoside, and coco glucoside.

Examples of Zwitterionic surfactants include those which can be broadly described as derivatives of aliphatic quaternary ammonium, phosphonium, and sulfonium compounds in which the aliphatic radicals can be straight chain or branched and wherein one of the aliphatic substituents contains from 8 to 18 carbon atoms and one contains an anionic water-solubilizing group such as carboxyl, sulfonate, sulfate, phosphate or phosphonate. Specific examples of suitable Zwitterionic surfactants include alkyl betaines, such as cocodimethyl carboxymethyl betaine, lauryl dimethyl carboxymethyl betaine, lauryl dimethyl alpha-carboxy-ethyl betaine, cetyl dimethyl carboxymethyl betaine, lauryl bis-(2-hydroxy-ethyl)carboxy methyl betaine, stearyl bis-(2-hydroxy-propyl)carboxymethyl betaine, oleyl dimethyl gamma-carboxypropyl betaine, and lauryl bis-(2-hydroxypropyl)alpha-carboxyethyl betaine, amidopropyl betaines, and alkyl sultaines, such as cocodimethyl sulfopropyl betaine, stearyldimethyl sulfopropyl betaine, lauryl dimethyl sulfoethyl betaine, lauryl bis-(2-hydroxy-ethyl)sulfopropyl betaine, and alkylamidopropylhydroxy sultaines.

Examples of amphoteric surfactants include derivatives of aliphatic secondary and tertiary amines in which the aliphatic radical can be straight chain or branched and wherein one of the aliphatic substituents contains from 8 to 18 carbon atoms and one contains an anionic water solubilizing group. Specific examples of suitable amphoteric surfactants include the alkali metal, alkaline earth metal, ammonium or substituted ammonium salts of alkyl amphocarboxy glycinates and alkyl amphocarboxypropionates, alkyl amphodipropionates, alkyl amphodiacetates, alkyl amphoglycinates, and alkyl amphopropionates, as well as alkyl iminopropionates, alkyl iminodipropionates, and alkyl amphopropylsulfonates, such as for example, cocoamphoacetate cocoamphopropionate, cocoamphodiacetate, lauroamphoacetate, lauroamphodiacetate, lauroamphodipropionate, lauroamphodiacetate, cocoamphopropyl sulfonate caproamphodiacetate, caproamphoacetate, caproamphodipropionate, and steoroamphoacetate.

An exemplary composition that includes the structured spherulite surfactant system includes a cationic surfactant at up to about 20 wt.%, more typically from 0.1 to 10 wt.%, and still more typically from 0.5 to 6 wt.%. The exemplary composition include the nonionic surfactant at up to about 20 wt.%, more typically from 0.5 to 10 wt.%, and still more typically from 1 to 6 wt.% of a nonionic surfactant. Furthermore, the exemplary composition includes the Zwitterinic or amphoteric surfactant at up to about 25 wt.%, more typically from 1 to 20 wt.%, and still more typically from 2 to 10 wt.%.

As previously mentioned, an exemplary spherulite structured system includes any surfactant blend that is manufactured by Rhodia and sold under the trademark Miracare^{®} SLB. Some particular systems include the surfactant blends sold under the trademark Miracare^{®} SLB-365™, which includes a mixture of anionic surfactant sodium trideceth sulfate, amphoteric surfactant sodium lauroamphoacetate, and nonionic surfactant cocomonoethanolamide. These surfactants will together form a spherulite structured system in an appropriate environment having a pH greater than 4 and less than 9, most preferably between 5 and 5.5. Accordingly, pH adjusting agents may be included in the formulation such as citric acid, succinic acid, phosphoric acid, sodium hydroxide, or sodium carbonate.

Furthermore, the packing parameter of the surfactant system should be approximately 1 in order for spherulite formation to occur instead of the formation of micelles or other structures. Salt concentration in the final formulation plays an important role on the packing parameter. Salt increases the surfactant packing and thus structures the surfactant blend in the final formulation. Thus, a suitable electrolyte may be included in the formulation such as salts of multivalent anions and/or cations, salts of monovalent cations with monovalent anions, and polyelectrolytes. According to an exemplary embodiment, the electrolyte or salt is present at a concentration ranging between 2.0 and 2.5 wt.%.

A personal care composition that includes, in part, a spherulite structured surfactant system such as the Miracare^{®} SLB-365™ blend can stably incorporate an effective combination of UVA and UVB sunscreens, although as will be discussed subsequently a solubilizer is also an important ingredient for satisfactory sunscreen incorporation and stability. The composition is able to remove contaminants and oil from the skin exterior surface while beneficially, generating plentiful generation of lather. Importantly, such a personal care composition enables the sufficient deposition of the combined UVA and UVB sunscreen compounds on the skin surface to provide a sustained SPF of 4 to 8 to the skin.

A solubilizer is included to homogenously incorporate the sunscreen compounds into the personal care formulation and to impart stability to the personal care composition over extended time and changes in temperature. Consequently, phase separation due to coalescence of sunscreen compounds and other otherwise insoluble components is not experienced. The solublizer is provided to maintain the composition as a stable, non-separating mixture of sunscreen components and surfactants over a long period of time even if the composition is subject to broad changes in temperature. Among suitable solubilizers are compounds selected from the group of polyethoxylated fatty acids or polyethoxylated fatty acid glycerides having between 5 and 30 moles of ethoxy units. Just a few examples include PEG-(5, 10, 20, 25, 30) hydrogenated tallow amine, PEG-15 hydrooxystearate, PEG-(6, 10) olive glycerides, PEG-8 propylene glycol cocoate, PEG-25 propylene glycol stearate, PEG-(5, 10, 15, 20) cocamide, and PEG-(5, 10, 15, 20) cocamine. According to a preferred embodiment, suitable solubilizers are more particularly selected from the group of polyethoxylated fatty acid glycerides. Just a few examples include PEG-(7, 30) glyceryl cocoate, PEG-(15, 20, 30) glyceryl isostearate, PEG-(8, 12, 15, 20, 23, 30) glyceryl laurate, PEG-(5, 10, 20, 25, 30) glyceryl stearate, PEG-28 glyceryl tallowate, and PEG-(5, 10, 20) glyceryl triisostearate.

UVA and UVB sunscreen compounds are known and may be selected according to their individual properties as desirable. The sunscreen compounds may be organic or inorganic, or a combination of both may be included. Sunscreens of use in the invention include UV absorbers or blockers. Some exemplary sunscreens are classified as para-amino benzoates, salicylates, cinnamates, and benzophenones, although this list is not to be considered as comprehensive. Some specific sunscreens that may be suitably used in conjunction with the spherulite surfactant system in a liquid soap, body wash, or other personal cleansing composition include p-aminobenzoic acid, its salts and its derivatives (ethyl, isobutyl, glyceryl esters; p-dimethylaminobenzoic acid); anthranilates (i.e., o-aminobenzoates; methyl, menthyl, phenyl, benzyl, phenylethyl, linalyl, terpinyl, and cyclohexenyl esters); salicylates (amyl, phenyl, benzyl, menthyl, glyceryl, and dipropylene glycol esters); cinnamic acid derivatives (methyl and benzyl esters, alpha-phenyl cinnamonitrile; butyl cinnamoyl pyruvate); dihydroxycinnamic acid derivatives (umbelliferone, methylumbelliferone, methylaceto-umbelliferone); trihydroxycinnamic acid derivatives (esculetin, methylesculetin, daphnetin, and the glucosides, esculin and daphnin); hydrocarbons (diphenylbutadiene, stilbene); dibenzalacetone and benzalacetophenone; naphtholsulfonates (sodium salts of 2-naphthol-3,3-disulfonic and of 2-naphthol-6,8-disulfonic acids); dihydroxynaphthoic acid and its salts; o- and p-hydroxybiphenyldisulfonates; coumarin derivatives (7-hydroxy, 7-methyl, 3-phenlyll); diazoles (2-acetyl-3-bromoindazole, phenyl benzoxazole, methyl naphthoxalole, various aryl benzothiazoles); Quinine salts (bisulfate, sulfate, chloride, oleate, and tannate); quinoline derivatives (8-hydroxyquinoline salts, 2-phenylquinoline); hydroxy- or methoxy substituted benzophenones; Uric and vilouric acids; Tannic acid and its derivatives (e.g., hexaethylether); (Butyl carbityl) (6-propyl piperonyl) ether; Hydroquinone; Benzophenones (oxybenzene, sulisobenzone, dioxybenzone, benzoresorcinol, 2,2',4,4'-Tetrahydroxybenzophenone, 2,2'-Dihydroxy-4,4'-dimethoxybenzophenone, octabenzone; 4-Isopropyhldibenzoylmethane; butylmethoxydibenzoylmethane; etocrylene; and 4-isopropyl-di-benzoylmethane; titanium dioxide, iron oxide, zinc oxide, and mixtures thereof. Other cosmetically-acceptable sunscreens and concentrations (percent by weight of the total cosmetic sunscreen composition) include diethanolamine methoxycinnamate (10% or less), elhyl-[bis(hydroxypropyl)]aminobenzoate (5% or less), glyceryl aminobenzoate (3% or less), 4-isopropyl dibenzoylmethane (5% or less), 4-methylbenzylidene camphor (6% or less), terephthalylidene dicamphor sulfonic acid (10% or less), and sulisobenzone (also called benzophenone-4, 10% or less).

For example, for a product marketed in the United States, preferred cosmetically-acceptable sunscreens and concentrations (reported as a percentage by weight of the total cosmetic sunscreen composition, and referring to the final percentage of the sunscreen after addition to the personal cleansing composition) include: aminobenzoic acid (also called para-aminobenzoic acid and PABA; 15% or less; a UVB absorbing organic sunscreen), avobenzone (also called butyl methoxy dibenzoylmethane; 3% or less, a UVA I absorbing organic sunscreen), cinoxate (also called 2-ethoxyethyl p-methoxycinnamate; 3% or less, a UVB absorbing organic sunscreen), dioxybenzone (also called benzophenone-8; 3% or less, a UVB and UVA II absorbing organic sunscreen), homosalate (15% or less, a UVB absorbing organic sunscreen), menthyl anthranilate (also called menthyl 2-aminobenzoate; 5% or less, a UVA II absorbing organic sunscreen), octocrylene (also called 2-ethylhexyl-2-cyano-3,3 diphenylacrylate; 10% or less, a UVB absorbing organic sunscreen), octyl methoxycinnamate (7.5% or less, a UVB absorbing organic sunscreen), octyl salicylate (also called 2-ethylhexyl salicylate; 5% or less, a UVB absorbing organic sunscreen), oxybenzone (also called benzophenone-3; 6% or less, a UVB and UVA II absorbing organic sunscreen), padimate O (also called octyl dimethyl PABA; 8% or less, a UVB absorbing organic sunscreen), phenylbenzimidazole sulfonic acid (water soluble; 4% or less, a UVB absorbing organic sunscreen), sulisobenzone (also called benzophenone-4; 10% or less, a UVB and UVA II absorbing organic sunscreen), titanium dioxide (25% or less, an inorganic physical blocker of UVA and UVB), trolamine salicylate (also called triethanolamine salicylate; 12% or less, a UVB absorbing organic sunscreen), and zinc oxide (25% or less, an inorganic physical blocker of UVA and UVB).

An exemplary liquid hand soap, body wash, or other personal cleansing composition contains at least one sunscreen active that is cinnamate (e.g., octylmethoxycinnamate (ethyl hexyl methoxycinnamate), (available under the tradename PARSOL MCX), oxybenzone (e.g., benzophenone-3 (2-hydroxy-4-mcthoxybenzophenone), avobenzone (4-tert-butyl-4'-methoxydibenzoylmethane or PARASOL 1789), octyl salicylate (2-ethylhexyl salicylate), octocrylene (2-ethylhexyl 2-cyano-3,3-diphenylacrylate), methyl anthranilate, and/or titanium dioxide, or combinations thereof.

An exemplary liquid hand soap, body wash, or other personal cleansing product also includes one or more polymers and/or thickeners such as clays, substituted or unsubstituted hydrocolloids, acrylates, and acrylates crosspolymers. Some examples of clays include bentonite, kaolin, montmorillonite, sodium magnesium silicate, hectorite, and magnesium aluminum silicate. Some examples of hydrocolloids in the unmodified form include agar, alginate, arabinoxylan, carrageenan, cellulose derivatives, curdlan, gelatin, gellan, guar gum, gum arabic, locust bean gum, pectin, starch, succinoglycan, and Xanthan gum. Some examples cellulose derivatives include carboxyalkyl cellulose, hydroxyalkyl cellulose, hydroxyalkyl alkyl cellulose, alkyl cellulose, hydroxy methyl cellulose, PG-hydroxyethyl cellulose, and quaternary ammonium derivatives of hydroxyethyl cellulose. Some examples of guar gum derivatives include quaternary ammonium derivatives of guar gum such as guar gum 2-Hydroxy-3-(Trimethylammonio) Propyl Ether Chloride (sold under the marks Jaguar C-17, Jaguar C-14S, and Jaguar Excel from Rhodia), and guar gum 2-hydroxypropyl 2-Hydroxy-3-(Trimethylammonio) Propyl Ether Chloride (manufactured and sold under the mark Jaguar C-162 from Rhodia), and hydroxypropyl derivatives of guars such as those sold under the marks Jaguar HP-8, Jaguar HP-105, Jaguar HP-60, and Jaguar HP-120 sold by Rhodia. Some examples of modified starches include sodium hydroxypropyl starch phosphate, and modified potato starch. Some examples of acrylate copolymers include acrylates/aminoacrylates/C10-30 alkyl PEG-20 itaconate copolymer, cationic polymers such as those sold under the marks Rheovis CSP, Rheovis CDE, Rheovis CDP from Ciba, polyacrylimidomethylpropane Sulfonate/Polyquatemium-4 sold under the mark Plexagel ASC from ISP, hydrohobically modified nonionic polyols such as those sold under the marks Acusol 880, Acusol 882 from Rohm & Haas, and PEG-150 distearate. In general, exemplary personal care compositions include, independently for each such ingredient, up to about 10 wt.%, typically from 0.1 to 5.0 wt.%, more typically from 0.5 to 4.0 wt.% of such other ingredients depending on the desired properties of the liquid hand soap, body wash or other personal cleansing composition.

### Example 1 and Comparative Example 1

A list of ingredients for one example of a personal cleansing composition is set forth in Table 1 below. Likewise, a list of ingredients for a comparative example of a personal cleansing composition that does not included a structured surfactant system is set forth in Table 2 below. Although the exemplary formula in Table I represents a personal cleansing composition, it is within the purview of the inventors for other similar personal cleansing formulas to be readily prepared that incorporate a structured surfactant system and combined UVA and UVB SPF agents. Shampoos, hand soaps, and face wash formulations are just a few examples of other exemplary personal cleansing formulations.

**Table 1 (Example 1)**

| | Ingredient | Trade Name | Activity (%) | Active Wt (%) | Blending Wt(%) |
|---|---|---|---|---|---|
| 1 | Water (D.I.) | Water | 100 | 49.08 | 48.91 |
| 2 | Guar Gum Derivative | Jaguar C-162 | 90 | 0.2 | 0.22 |
| 3 | Tetrasodium EDTA | Versenc 100 | 40 | 0.1 | 0.25 |
| 4 | Glycerin | Glycerin | 100 | 2 | 2.00 |
| 5 | Surfactant Blend | Miracare SLB-365/G | 100 | 35 | 35.00 |
| 6A | PEG-7 Glyceryl Cocoate | Cetiol HE | 100 | 2.5 | 2.50 |
| 6B | Octocrylene | NEO Heliopan 303 | 100 | 5 | 5.00 |
| 6C | Avobenzone | NEO Heliopan 357 | 100 | 3 | 3.00 |
| 7 | Sodium Chloride | Sodium Chloride | 100 | 2.5 | 2.50 |
| 8 | Citric Acid (Anhydrous) | Citric Acid | 100 | 0.62 | 0.62 |

**Table 2 (Comparative Example 1)**

| | Ingredients | Trade Name | Activity (%) | Active Wt (%) | Blending Wt (%) |
|---|---|---|---|---|---|
| I | Water (D.I.) | Water | 100 | 74.95 | 46.99 |
| 2 | Guar Gum Derivative | Jaguar C-162 | 90 | 0.2 | 0.22 |
| 3 | Tetrasodium EDTA | Versene 100 | 40 | 0.1 | 0.25 |
| 4 | Glycerin | Glycerin | 100 | 2 | 2.00 |
| 5 | Cocamidopropyl Betaine | Mackam 35-HP | 30 | 5.2 | 17.33 |
| 6 | Sodium Laureth Sulfate | Calfoam ES-302 | 26 | 5.5 | 21.15 |
| 7A | PEG-7 Glyceryl Cocoate | Cetiol HE | 100 | 2.5 | 2.50 |
| 7B | Octocrylene | NEO Heliopan 303 | 100 | 5 | 5.00 |
| 7C | Avobenzone | NEO Heliopan 357 | 100 | 3 | 3.00 |
| 7D | Sorbitan Monooleate | Span 80 | 100 | 1.1 | 1.10 |
| 8 | Sodium Chloride | Sodium Chloride | 100 | 0.4 | 0.40 |
| 9 | Citric Acid (Anhydrous) | Citric Acid | 100 | 0.05 | 0.05 |

The exemplary formula set forth in Table 1 may be prepared by adding ingredients 1 to 5 in sequence. The mixture may then be heated to between 110 and 130 °F (43 to 49 °C). Ingredients 6A and 6B may be premixed separate from the heated mixture, and then heated to about 160 °F (72 °C). Ingredient 6C may be slowly added to the premix while heating until it dissolves and the premix becomes a clear yellow solution. The premix may then be added to the mixture of ingredients 1 to 4, and the heat is turned off for the addition of ingredients 7 and 8.

The comparative formula set forth in Table 2 may be prepared similarly by adding ingredients 1 to 6 in sequence. The mixture may then be heated to between 110 and 130 °F (43 to 49 °C). Ingredients 7A and 7B may be premixed separate from the heated mixture, and then heated to about 160 °F (72 °C). Ingredient 7C may be
slowly added to the premix while heating until it dissolves and the premix becomes a clear yellow solution. Ingredient 7D is then added to the premix. The premix may then be added to the mixture of ingredients 1 to 4, and the heat is turned off for the addition of ingredients 8 and 9.

The pH of the solutions from the exemplary formula and the comparative formula at room temperature are similar (5.2 for the exemplary formula, 6.25 for the comparative formula). However, the viscosity for the solution from the exemplary formula measures 17156 cp at room temperature, while the viscosity for the solution from the comparative formula measures 7778 cp.

In addition, the personal cleansing compositions produced using the exemplary and comparative formulas may be compared for their lathering properties, as well as their ability to deposit sunscreen compounds and to retain their stability over time. The comparative formula produces a personal cleansing composition that produced a poor lather, exhibits phase separation after centrifuging at 15000 rpm for 30 minutes at room temperature, and provides no SPF after rinsing the personal cleansing composition. In contrast, the exemplary formula produces a good lathering personal cleansing composition that maintains its stability without phase separation after centrifuging at 1500 rpm for 30 minutes at room temperature.

Furthermore, the personal cleansing composition from the exemplary formula deposits sunscreen compounds, imparting a sustained SPF of approximately 5 after rinsing the personal cleansing composition. For example, in vitro tests were performed on skin samples that were incubated at constant relative humidity. The cleansing formulations of both Example 1 and Comparative Example 1 were diluted with water to typical cleansing dilution and then applied to different skin samples. The skin was rinsed eight times with water in horizontal and vertical directions, including gentle rubbing with gloved fingers. SPF measurements on the air dried skin samples were performed using a spectrophotometer, finding a SPF of 5 for the samples washed with the formulation of Example 1. In contrast, the skin samples washed with the formulation of Comparative Example 1 did not have a measurable SPF.

### Comparative Example 2

A personal cleansing composition formulation similar to that of Table 1, including the structured surfactant blend (Ingredient 4) but without any PEG-7 glyceryl cocoate or other solubilizer (Ingredient 6A), may be prepared following equivalent procedural steps. After combining all of the ingredients, much of the sunscreen (Ingredients 6B and 6C) remains undissolved in the formulation. After raising the temperature to 100 °F (38 °C) and mixing for 30 minutes, much of the sunscreen
still remains undissolved.

### Comparative Example 3

A personal cleansing composition formulation similar to that of Table 1, but replacing the PEG-7 glyceryl cocoate (Ingredient 6A) with a different solubilizer, capric triclyceride (blending weight 2.5 g), may be prepared following equivalent procedural steps. Capric triglyceride is recognized as a good solubilizer for both of the sunscreens (Ingredients 6B and 6C). More particularly, both avobenzone and octocrylene are known to be miscible with capric triglyceride, with avobenzone being miscible at a concentration of up to 16 wt.%. However, after combining all of the ingredients the final personal cleansing composition formulation has a low viscocity and exhibits phase separation after centrifuging the formulation for 30 minutes at 15000 rpm.

## Claims

1. A homogeneous and shelf stable personal cleansing composition, comprising:
a structured surfactant system comprising a blend of surfactants that form spherulite structures in an aqueous medium;
water;
at least one sunscreen compound; and
a solubilizer comprising one or more polyethoxylated fatty acid, or polyethoxylated fatty acid glyceride, having between 5 to 30 moles of ethoxy units;
wherein the personal cleansing composition deposits the at least one sunscreen compound onto skin in a manner whereby the sunscreen compound imparts to the skin a sun protection factor of at least 4 after rinsing the personal cleansing composition.

2. The personal cleansing composition according to claim 1, wherein the solubilizer comprises one or more polyethoxylated fatty acid glycerides selected from the group consisting of PEG-(7, 30)
glyceryl cocoate, PEG-(15, 20, 30) glyceryl isostearate, PEG-(8, 12, 15, 20, 23, 30) glyceryl laurate, PEG-(5, 10, 20, 25, 30) glyceryl stearate, PEG-28 glyceryl tallowate, and PEG-(5, 10, 20) glyceryl triisostearate.

3. The personal cleansing composition according to claim 2, wherein the solubilizer comprises PEG-7 glyceryl cocoate.

4. The personal cleansing composition according to any preceding claim, wherein the structured surfactant system comprises an anionic surfactant, a nonionic surfactant, and an amphoteric surfactant.

5. The personal cleansing composition according to claim 4, wherein the anionic surfactant comprises sodium trideceth sulfate, the amphoteric surfactant comprises sodium lauroamphoacetate and the nonionic surfactant comprises cocomonoethanolamide.

6. The personal cleansing composition according to any preceding claim, further comprising an electrolyte at a concentration ranging between 0,5 wt.% and 6 wt.%.

7. The personal cleansing composition according to any preceding claim, further comprising a thickener.

8. The personal cleansing composition according to claim 7, wherein the thickener is selected from the group consisting of clays, substituted or unsubstituted hydrocolloids, acrylates, and acrylates crosspolymers.

9. The personal cleansing composition according to claim 8, wherein the thickener comprises a quaternary ammonium derivative of guar gum or a hydroxypropyl derivative of guar gum.

10. Composition for use in a method of cleansing and providing sun protection factor to skin, the method comprising:
applying to the skin a homogeneous and shelf stable personal cleansing composition, comprising:
a structured surfactant system comprising a blend of surfactants that form spherulite structures in an aqueous medium;
water;
at least one sunscreen compound; and
a solubilizer comprising one or more polyethoxylated fatty acid, or polyethoxylated fatty acid glyceride, having between 5 to 30 moles of ethoxy units;
rinsing the personal cleansing composition from the skin, whereby a sufficient amount of at least one sunscreen compound remains deposited onto the skin to impart to the skin a sun protection factor of at least 4 after rinsing the personal cleansing composition.

11. Composition for use according to claim 10, wherein the solubilizer applied to the skin with the personal cleansing composition comprises PEG-7 glyceryl cocoate.

12. Composition for use according to claim 10 or 11, wherein the structured surfactant system applied to the skin with the personal cleansing composition comprises an anionic surfactant, a nonionic surfactant, and an amphoteric surfactant.

13. Composition for use according to claim 10, wherein the personal cleansing composition applied to the skin further comprises an electrolyte at a concentration ranging between 0,5 wt.% and 6 wt.%.

14. Composition for use according to claim 10, wherein the personal cleansing composition applied to the skin further comprises a thickener.

## Patentansprüche

1. Homogene und haltbare Körperreinigungszusammensetzung, Folgendes umfassend:
ein strukturiertes Tensidsystem, umfassend eine Mischung aus Tensiden, die in einem wässrigen Medium Sphärolithstrukturen ausbilden;
Wasser;
wenigstens eine Sonnenschutzverbindung; und
einen Lösungsvermittler, umfassend eine/ein oder mehrere polyethoxylierte Fettsäure oder polyethoxyliertes Fettsäureglycerid mit zwischen 5 und 30 Mol Ethoxy-Einheiten;
wobei die Körperreinigungszusammensetzung die wenigstens eine Sonnenschutzverbindung auf eine Weise auf der Haut ablagert, wodurch die Sonnenschutzverbindung der Haut nach dem Abspülen der Körperreinigungszusammensetzung einen Lichtschutzfaktor von wenigstens 4 verleiht.

2. Körperreinigungszusammensetzung nach Anspruch 1, wobei der Lösungsvermittler ein oder mehrere polyethoxylierte Fettsäureglyceride umfasst, ausgewählt aus der Gruppe bestehend aus PEG-(7, 30)-Glycerylcocoat, PEG-(15, 20, 30)-Glycerylisostearat, PEG-(8, 12, 15, 20, 23, 30)-Glyceryllaurat, PEG-(5, 10, 20, 25, 30)-Glycerylstearat, PEG-28-Glyceryltallowat und PEG-(5, 10, 20)-Glyceryltriisostearat.

3. Körperreinigungszusammensetzung nach Anspruch 2, wobei der Lösungsvermittler PEG-7-Glycerylcocoat umfasst.

4. Körperreinigungszusammensetzung nach einem der vorhergehenden Ansprüche, wobei das strukturierte Tensidsystem ein anionisches Tensid, ein nichtionisches Tensid und ein amphoteres Tensid umfasst.

5. Körperreinigungszusammensetzung nach Anspruch 4, wobei das anionische Tensid Natrium-Tridecethsulfat umfasst, das amphotere Tensid Natrium-Lauroamphoacetat umfasst und das nichtionische Tensid Cocomonoethanolamid umfasst.

6. Körperreinigungszusammensetzung nach einem der vorhergehenden Ansprüche, ferner umfassend einen Elektrolyten in einer Konzentration im Bereich zwischen 0,5 Gew.-% und 6 Gew.-%.

7. Körperreinigungszusammensetzung nach einem der vorhergehenden Ansprüche, ferner einen Verdicker umfassend.

8. Körperreinigungszusammensetzung nach Anspruch 7, wobei der Verdicker ausgewählt ist aus der Gruppe bestehend aus Tonarten, substituierten oder unsubstituierten Hydrokolloiden, Acrylaten und Acrylaten-Crosspolymeren.

9. Körperreinigungszusammensetzung nach Anspruch 8, wobei der Verdicker ein quaternäres Ammoniumderivat von Guargummi oder ein Hydroxypropylderivat von Guargummi umfasst.

10. Zusammensetzung zum Gebrauch in einem Verfahren zum Reinigen und Bereitstellen eines Lichtschutzfaktors an Haut, wobei das Verfahren Folgendes umfasst:
Auftragen einer homogenen und haltbaren Körperreinigungszusammensetzung auf die Haut, Folgendes umfassend:
ein strukturiertes Tensidsystem, umfassend eine Mischung aus Tensiden, die in einem wässrigen Medium Sphärolithstrukturen ausbilden;
Wasser;
wenigstens eine Sonnenschutzverbindung; und
einen Lösungsvermittler, umfassend eine/ein oder mehrere polyethoxylierte Fettsäure oder polyethoxyliertes Fettsäureglycerid mit zwischen 5 und 30 Mol Ethoxy-Einheiten;
Abspülen der Körperreinigungszusammensetzung von der Haut, wodurch eine ausreichende Menge von wenigstens einer Sonnenschutzverbindung auf der Haut abgelagert bleibt, um der Haut einen Lichtschutzfaktor von wenigstens 4 nach dem Abspülen der Körperreinigungszusammensetzung zu verleihen.

11. Zusammensetzung zum Gebrauch nach Anspruch 10, wobei der mit der Körperreinigungszusammensetzung auf die Haut aufgetragene Lösungsvermittler PEG-7-Glycerylcocoat umfasst.

12. Zusammensetzung zum Gebrauch nach Anspruch 10 oder 11, wobei das mit der Körperreinigungszusammensetzung auf die Haut aufgetragene strukturierte Tensidsystem ein anionisches Tensid, ein nichtionisches Tensid und ein amphoteres Tensid umfasst.

13. Zusammensetzung zum Gebrauch nach Anspruch 10, wobei die auf die Haut aufgetragene Körperreinigungszusammensetzung einen Elektrolyten in einer Konzentration im Bereich zwischen 0,5 Gew.-% und 6 Gew.-% umfasst.

14. Zusammensetzung zum Gebrauch nach Anspruch 10, wobei die auf die Haut aufgetragene Körperreinigungszusammensetzung ferner einen Verdicker umfasst.

## Revendications

1. Composition nettoyante pour les personnes, homogène et stable au stockage, contenant :
un système tensioactif structuré comprenant un mélange de tensioactifs formant des structures sphérulites dans un milieu aqueux ;
de l'eau ;
au moins un composé écran solaire ; et
un solubilisant comprenant au moins un acide gras polyéthoxylé, ou un glycéride d'acide gras polyéthoxylé, comprenant entre 5 et 30 moles d'unités d'éthoxy ;
la composition nettoyante pour les personnes déposant l'au moins un composé écran solaire sur la peau de sorte que le composé écran solaire apporte à la peau un facteur de protection solaire d'au moins 4 après rinçage de la composition nettoyage pour les personnes.

2. Composition nettoyante pour les personnes selon la revendication 1, dans laquelle le solubilisant contient au moins un glycéride d'acide polyéthoxylé choisis dans le groupe constitué du cocoate de glycéryle PEG-(7,30), de l'isostéarate de glycéryle PEG-(15,20,30), du laurate de glycéryle PEG-(8,12,15,20,23,30), du stéarate de glycéryle PEG-(5,10,20,25,30), du tallowate de glycéryle PEG-28 et du triisostéarate de glycéryle PEG-(5,10,20).

3. Composition nettoyante pour les personnes selon la revendication 2, dans laquelle le solubilisant contient du cocoate de glycéryle PEG-7.

4. Composition nettoyante pour les personnes selon l'une quelconque des revendications précédentes, dans laquelle le système tensioactif structuré comprend un tensioactif anionique, un tensioactif non-ionique et un tensioactif amphotère.

5. Composition nettoyante pour les personnes selon la revendication 4, dans laquelle le tensioactif anionique comprend du tridéceth sulfate de sodium, le tensioactif amphotère comprend du lauroamphoacétate de sodium, et le tensioactif non-ionique comprend du cocomonoéthanolamide.

6. Composition nettoyante pour les personnes selon l'une quelconque des revendications précédentes, contenant en outre un électrolyte à une concentration entre 0,5 % et 6 % en poids.

7. Composition nettoyante pour les personnes selon l'une quelconque des revendications précédentes, contenant en outre un épaississant.

8. Composition nettoyante pour les personnes selon la revendication 7, dans laquelle l'épaississant est choisi dans le groupe constitué d'argiles, d'hydrocolloïdes substitués ou non, d'acrylates et de polymères croisés acrylates.

9. Composition nettoyante pour les personnes selon la revendication 8, dans laquelle l'épaississant contient un dérivé ammonium quaternaire de la gomme de guar ou un dérivé hydroxypropyle de la gomme de guar.

10. Composition destinée à être utilisée dans un procédé de nettoyage et de fourniture d'un facteur de protection solaire pour la peau, le procédé comprenant :
l'application sur la peau d'une composition nettoyante pour les personnes, homogène et stable au stockage, contenant :
un système tensioactif structuré comprenant un mélange de tensioactifs formant des structures sphérulites dans un milieu aqueux ;
de l'eau ;
au moins un composé écran solaire ; et
un solubilisant comprenant au moins un acide gras polyéthoxylé, ou un glycéride d'acide gras polyéthoxylé, comprenant entre 5 et 30 moles d'unités d'éthoxy ;
le rinçage de la composition nettoyante pour les personnes, une quantité suffisante d'au moins un composé écran solaire restant déposé sur la peau pour lui conférer un facteur de protection solaire d'au moins 4 après rinçage de la composition nettoyante pour les personnes.

11. Composition destinée à être utilisée selon la revendication 10, dans laquelle le solubilisant appliqué sur la peau avec la composition nettoyante pour les personnes contient du cocoate de glycéryle PEG-7.

12. Composition destinée à être utilisée selon la revendication 10 ou 11, dans laquelle la composition nettoyante pour les personnes comprend un tensioactif anionique, un tensioactif non-ionique et un tensioactif amphotère.

13. Composition destinée à être utilisée selon la revendication 10, dans laquelle la composition nettoyante pour les personnes, appliquée sur la peau, contient en outre un électrolyte à une concentration entre 0,5 % et 6 % en poids.

14. Composition destinée à être utilisée selon la revendication 10, dans laquelle la composition nettoyante pour les personnes contient en outre un épaississant.
